# EUROPÄISCHE PATENTSCHRIFT

(11) **EP 3 897 514 B1**
(45) Veröffentlichungstag und Bekanntmachung des Hinweises auf die Patenterteilung: **09.09.2026**
(21) Anmeldenummer: 19805176.5
(22) Anmeldetag: 12.11.2019
(51) Int. Cl.: A61K 8/03, A61K 8/31, A61K 8/37, A61K 8/92, A61Q 5/12

(54) **SILIKON-FREIE ZWEIPHASEN-HAARKUR**
SILICONE-FREE BIPHASIC HAIR TREATMENT
SOIN CAPILLAIRE À DEUX PHASES EXEMPT DE SILICONE

(30) Priorität: 17.12.2018 DE 102018221949
(43) Veröffentlichungstag der Anmeldung: 27.10.2021
(73) Patentinhaber: Henkel AG & Co. KGaA, 40589 Düsseldorf (DE)
(72) Erfinder: HARTWICH, Christa, 25337 Elmshorn (DE); VON BORSTEL, Christin, 21706 Drochtersen (DE)
(86) Internationale Anmeldenummer: PCT/EP2019/080933
(87) Internationale Veröffentlichungsnummer: WO 2020/126219

(56) Entgegenhaltungen:
- FR-A1- 2 971 421
- FR-A1- 3 038 514
- US-A1- 2004 241 200
- US-B2- 10 016 348

## Beschreibung

Die Anmeldung beschreibt Haarbehandlungsmittel in Form eines Zweiphasensystems mit einer Öl- und einer Wasserphase in Form zweier separater, optisch erkennbarer kontinuierlicher Phasen mit gemeinsamer horizontaler Phasengrenze, die frei von Silikonen sind und die in der Ölphase einen hohen Anteil an Isoparaffinen mit 9 bis 17 Kohlenstoffatomen aufweisen.

Die Anmeldung beschreibt weiterhin ein Verfahren zur Haarkonditionierung unter Verwendung der Haarbehandlungsmittel sowie die Verwendung der Haarbehandlungsmittel zur Verbesserung spezifischer Pflegeeigenschaften von Haaren.

Zweiphasen-Haarkonditioniermittel sind seit langem aus dem Stand der Technik bekannt und werden aufgrund ihrer ansprechenden Optik bei den Verbrauchern sehr geschätzt. Üblicherweise handelt es sich dabei um leave-on-Produkte, die vorzugsweise unmittelbar nach kurzzeitiger Durchmischung der beiden Phasen auf die Haare gebracht (vorzugsweise gesprüht) werden und diese pflegen.

In der Ölphase enthalten die allermeisten dieser Produkte cyclische Silikone.

Aus ökologischen Gesichtspunkten sowie aus Gründen der höheren Verbraucherakzeptanz besteht aber das Bedürfnis nach möglichst Silikon-freien, gut pflegenden Haarbehandlungsmitteln.

In der Vergangenheit wurden bereits entsprechende Produkte vorgeschlagen, in denen (cyklische) Silikone teilweise oder vollständig durch andere Fettphasenbestandteile ersetzt wurden:
WO 2010/069995 beschreibt Zweiphasenprodukte mit hohem Polyolgehalt in der Wasserphase, die in der Ölphase mindestens 5 Gew.-% eines Nicht-Silikonöls (beispielsweise Paraffine und/oder Isoparaffine) enthalten.
Als Ersatz für cyklische Silikone in kosmetischen Hautbehandlungsmitteln wurde in WO 2004/103308 eine Mischung aus Isododecan und einem spezifischen Ester vorgeschlagen und ausgeführt, dass die Flüchtigkeit und Verteilbarkeit dieser Mischung in etwa der von cyklischen Silikonen entspricht.Außerdem wird in FR 3038514 eine kosmetische, transparente Zweiphasen-Zusammensetzung beschrieben, die eine Glycerin-Wasserphase und eine silikonfreie Ölphase, umfassend ein pflanzliches Öl, zwei Ester und Triheptanoin.
US 10016348 betrifft transparente Zweiphasen-Zusammensetzungen, die APG-Fettsäureester und vernetzte Alkane enthalten. Die Zusammensetzungen können optional Silikone enthalten.
US 2004/241200 betrifft Cyclomethicon-freie Zusammensetzungen, die Neopentyl polyol polyester und Isoalkane enthalten.

Hinsichtlich mehrerer Haarpflegeaspekte besteht dennoch Verbesserungsbedarf. Darüber hinaus werden von den Verbrauchern gut pflegende Zusammensetzungen bevorzugt, die wenig komplex sind, einen hohen Anteil nachwachsender Rohstoffe sowie einen möglichst hohen Wasseranteil aufweisen.

Der vorliegenden Anmeldung lag die Aufgabe zugrunde, Silikon-freie Haarbehandlungsmittel in Form von Zweiphasensystemen mit hoher Pflegeleistung bereitzustellen, die eine optimale Balance zwischen langanhaltender und nachhaltiger Pflege sowie guter Verträglichkeit gewährleisten. Die Haarbehandlungsmittel sollen bei gleichbleibender Pflegeleistung einen möglichst hohen Wasseranteil aufweisen und insbesondere vorher geschädigten und/oder trockenen und/oder gesplissten Haaren bis in Spitzen wieder mehr Weichheit, Glanz und Geschmeidigkeit sowie verbesserte Entwirrbarkeit und Kämmbarkeit verleihen.

Ein weiteres Ziel bestand darin Zweiphasen-Haarbehandlungsmittel zur Verfügung zu stellen, die auch nach regelmäßiger Anwendung keine Beschwerung der Haare verursachen und die Haare leichter form- und frisierbar zu machen.

Es wurde festgestellt, dass durch Kombination spezieller Isoparaffine, Ester und pflanzlicher Öle in der Ölphase sowie einem hohen Wasseranteil in der Wasserphase Zweiphasen-Haarbehandlungsmittel bereitgestellt werden können, die die zuvor genannten Anforderungen in hohem Maße erfüllen.

Die Anwesenheit (cyklischer) Silikone ist weder für die Stabilität noch für die Pflegeeigenschaften derartiger Haarbehandlungsmittel erforderlich, denn diese liegen in Form temperatur- und lagerstabiler Zweiphasensystemene mit kontinuierlicher Öl- und kontinuierlicher Wasserphase vor, wobei sich die Phasen rasch und gründlich durch- und wieder entmischen lassen.

Darüber hinaus weisen die Haarbehandlungsmittel auf damit behandelten Haaren einen nachweisbaren Konditionierungsvorteil auf. Insbesondere die Kämmbarkeit, der Haarglanz und die Frisier- und Stylingeigenschaften insbesondere vorher strapazierter und/oder geschädigter Haare konnten signifikant verbessert werden. Doch auch die "anti-frizz"-Wirkung derartiger Haarbehandlungsmittel wurde in einem Verbrauchertest hervorragend bewertet.

Mit erfindungsgemäßen Mitteln behandelte Haare wiesen insgesamt ein gesünderes optisches Erscheinungsbild auf.

Ein erster Gegenstand der Erfindung ist ein Haarbehandlungsmittel in Form eines Zweiphasensystems mit zwei separaten, optisch erkennbaren kontinuierlichen Phasen mit gemeinsamer horizontaler Phasengrenze, enthaltend
- bezogen auf das Gesamtgewicht des Haarbehandlungsmittels - eine Wasserphase (I) in einem Gewichtsanteil von mindestens 85 Gew.-% und eine Ölphase (II) in einem Gewichtsanteil von höchstens 15 Gew.-%, wobei
- die Ölphase (II) - bezogen auf ihr Gesamtgewicht - eine Mischung aus
   i) 20 bis 40 Gew.-% mindestens eines Isoparaffins mit 9 bis 17 Kohlenstoffatomen,
   ii) 45 bis 65 Gew.-% mindestens zweier unterschiedlicher Mono- und/oder Diester von linearen oder verzweigten, gesättigten oder ungesättigten C₄-C₁₂-Carbonsäuren mit linearen oder verzweigten, gesättigten oder ungesättigten C₄-C₂₄-Mono- oder Dialkoholen, und
   iii) 5 bis 20 Gew.-% mindestens einems pflanzlichen Öls enthält, und
- die Haarbehandlungsmittel frei von Silikonverbindungen sind.

Die erfindungsgemäßen Haarbehandlungsmittel umfassen zwei optisch getrennte Phasen, die durch Schütteln homogenisiert werden können, und die sich im Ruhezustand wieder in zwei separate, optisch erkennbare kontinuierliche Phasen mit gemeinsamer horizontaler Phasengrenze trennen.

Optimalerweise weisen die erfindungsgemäßen Haarbehandlungsmittel innerhalb von zehn Stunden, vorzugsweise innerhalb von sieben Stunden, besonders bevorzugt innerhalb von fünf Stunden, ganz besonders bevorzugt innerhalb von drei Stunden und insbesondere innerhalb von einer Stunde optisch wieder ihren Ausgangszustand mit horizontaler Phasengrenze auf.

Für eine schnelle Durch- und Entmischung der beiden Phasen kann es von Vorteil sein, wenn die Wasserphase (I) und die Ölphase (II) geringere Viskositätswerte aufweisen. Unter "geringeren Viskositätswerten" sind im Rahmen der vorliegenden Erfindung vorzugsweise Viskositätswerte der Wasserphase (I) und der Ölphase (II) von jeweils bis zu maximal 1000 mPas, mehr bevorzugt 600 mPas und insbesondere 400 mPas zu verstehen (gemessen bei 20°C mit einem Brookfield-Viskosimeter DV-II, Spindel 2 bei 20 UpM).

Für eine bessere Erkennbarkeit der Durch- und Entmischung der beiden Phasen kann es weiterhin von Vorteil sein, wenn die Wasserphase (I) transparent ist.

Unter dieser Voraussetzung lässt sich eine ausreichende Durchmischung der beiden Phasen vorzugsweise daran erkennen, dass nur noch eine einzige Phase sichtbar ist, die opak bis milchig trüb sein kann.

Insbesondere bevorzugt ist mindestens eine der beiden Phasen (I) und (II) transparent.

Unter "Transparenz" wird im Rahmen der vorliegenden Erfindung verstanden, dass die Wasserphase (I) und/oder die Ölphase (II) im Ruhezustand bevorzugt einen NTU-Wert (Nephelometric Turbidity Unit) von maximal 100, vorzugsweise von maximal 75, mehr bevorzugt von maximal 50 und insbesondere von maximal 25 aufweisen (gemessen beispielsweise mit einem Turbidimeter des Typs Turbiquant^{®} von der Firma Merck).

In einer bevorzugten Ausführungsform ist die Wasserphase (I) transparent und die Ölphase (II) der Pflegephase milchig-cremig.

Ebenfalls zur besseren Visualisierung kann eine der beiden Phasen (I) oder (II), vorzugsweise die Wasserphase (I), mit einem kosmetisch akzeptablen Farbstoff angefärbt sein.

Es ist weiterhin wünschenswert und bevorzugt, dass die erfindungsgemäßen Haarbehandlungsmittel versprühbar sind.

In versprühbarer Form eignen sich die erfindungsgemäßen Haarbehandlungsmittel für die Applikation aus einem Pumpspender. Vorteilhaft an einer solchen Applikationsform ist die einfache, saubere und zeitsparende Handhabbarkeit, denn die Haarbehandlungsmittel lassen sich aus einem geeigneten Pumpspender durch einfache Betätigung eines Pumpventils als feiner Sprühnebel verteilen und erreichen alle Bereiche der Haare.

Die Hände kommen auf diese Weise nicht mit dem Mittel in Berührung und müssen nach der Anwendung des Mittels nicht gereinigt werden.

Für eine solche Applikationsform weisen die anwendungsbereiten (d.h. die durch Schütteln homogenisierten) erfindungsgemäßen Haarbehandlungsmittel bevorzugt eine Viskosität von maximal 1000 mPas, vorzugsweise von maximal 800 mPas, mehr bevorzugt von maximal 600 mPas und insbesondere von maximal 500 mPas (jeweils gemessen bei 20°C mit einem Brookfield-Viskosimeter DV-II, Spindel 2 bei 20 UpM) auf.

Ebenfalls bevorzugt für eine solche Applikationsform ist, dass die erfindungsgemäßen Haarkonditioniermittel frei von Treibmitteln sind.

Unter "frei von Silikonverbindungen" wird im Rahmen der vorliegenden Erfindung verstanden, dass den Haarbehandlungsmitteln keine freien Silikonverbindungen hinzugefügt werden.

Für den Fall, dass gegebenenfalls fakultativ in den Haarbehandlungsmitteln enthaltene Handelsprodukte geringe Mengen an Silikonverbindungen umfassen, ist unter "frei von Silikonverbindungen" zu verstehen, dass die Haarbehandlungsmittel (bezogen auf ihr Gesamtgewicht) vorzugsweise maximal 0,01 Gew.-%, mehr bevorzugt maximal 0,005 und besonders bevorzugt maximal 0,001 Gew.-% Silikonverbindungen enthalten.

Ganz besonders bevorzugt werden in den Haarbehandlungsmitteln ausschließlich Handelsprodukte verwendet, die keine Silikonverbindungen enthalten.

In einer bevorzugten Ausführungsform enthalten die erfindungsgemäßen Haarbehandlungsmittel
a) die Wasserphase (I) in einem Gewichtsanteil von mindestens 88 Gew.-% am Gesamtgewicht des Haarbehandlungsmittels und
b) die Ölphase (II) in einem Gewichtsanteil von höchstens 12 Gew.-% am Gesamtgewicht des Haarbehandlungsmittels.

Es ist im Rahmen der vorliegenden Erfindung weiterhin bevorzugt, wenn die Wasserphase (I) - bezogen auf ihr Gesamtgewicht - mindestens 85 Gew.-%, bevorzugt mindestens 90 Gew.-% und insbesondere mindestens 95 Gew.-% Wasser enthält.

Es wurde gefunden, dass sich besonders mittelkettige Isoparaffine i) problemlos in erfindungsgemäße Zweiphasen-Haarpflegemittel einarbeiten lassen.

Gegenüber Silikonen in entsprechenden Zweiphasen-Haarpflegemitteln weisen mittelkettige Isoparaffine i) eine in etwa gleichwertige haarpflegende Wirkung auf.

Isoparaffine mit 9 bis 17 Kohlenstoffatome i) eignen sich als Silikonersatzstoffe in Haarpflegemitteln aufgrund ihres Vermögens, den Haaren Glanz und Weichheit zu verleihen, ohne bei regelmäßiger Anwendung eine Beschwerung oder Nachfettung der Haare zu verursachen.

Unter geeigneten mittelkettigen Isoparaffinen i) werden vorzugsweise Isoparaffine mit 9 bis 17 Kohlenstoffatomen wie Isodecan, Isoundecan, Isododecan, Isotridecan, Isotetradecan sowie Mischungen dieser Isoparaffine verstanden.

Es können einzelne dieser Isoparaffine oder Gemische von zwei oder mehr Isoparaffinen eingesetzt werden. Als für die kosmetische Wirkung in den erfindungsgemäßen Haarbehandlungsmitteln besonders vorteilhaft hat sich der Einsatz von Isoparaffinen aus der Gruppe Isoundecan, Isododecan, Isotridecan erwiesen.

In einer weiteren bevorzugten Ausführungsform enthalten die erfindungsgemäßen Haarbehandlungsmittel als Isoparaffin i) Isodecan, Isoundecan, Isododecan, Isotridecan, Isotetradecan sowie Mischungen davon.

Insbesondere bevorzugt innerhalb dieser Ausführungsform sind erfindungsgemäße Haarbehandlungsmittel, die Isoundecan, Isododecan und/oder Isotridecan enthalten.

Der Gewichtsanteil des Isoundecans, Isododecans, Isotridecans am Gesamtgewicht aller in der Ölphase (II) eingesetzten Isoparaffine i) beträgt vorzugsweise > 60 Gew.-%, mehr bevorzugt > 70 Gew.-%, besonders bevorzugt > 80 Gew.-% und insbesondere bevorzugt > 90 Gew.-%.

Als Beispiele für besonders geeignete Isoparaffine i) seien die im Handel erhältlichen Produkte Purolan^{®} IDD (INCI-Bezeichnung: Isododecane), Pioneer^{®} 2094 und KC Solvent^{®} 130 (INCI-Bezeichnung: Isoundecane, Isododecane) genannt.

Geeignete Isoparaffine i) können in der Ölphase (II) der erfindungsgemäßen Mittel auch als Mischung mit einem oder mehreren Ester(n) ii) eingesetzt werden, beispielsweise als Handelsprodukt Lexfeel^{®} D5 (erhältlich von der Firma Inolex; INCI-Bezeichnung: Neopentyl Glycol Diheptanoate, Isododecane)
Das oder die Isoparaffin(e) i) kann (können) in den Ölphase (II) der erfindungsgemäßen Haarbehandlungsmittel bevorzugt in einem Gewichtsanteil von jeweils 22 bis 38 Gew.-%, besonders bevorzugt 25 bis 35 Gew.-% und insbesondere 27 bis 33 Gew.-% am Gesamtgewicht der Ölphase (II) eingesetzt werden.

Zur Erhöhung der Pflegewirkung enthalten die erfindungsgemäßen Haarbehandlungsmittel weiterhin mindestens zwei unterschiedliche Mono- und/oder Diester ii) linearer oder verzweigter, gesättigter oder ungesättigter C₄-C₁₂-Carbonsäuren mit linearen oder verzweigten, gesättigten oder ungesättigten C₄-C₂₄-Mono- oder Dialkoholen.

Geeignete Ester ii) müssen sich gut mit den Isoparaffinen i) und dem mindestens einen pflanzlichen Öl iii) vermischen lassen und als Mischung auf den Haaren eine hervorragende Verteilbarkeit und Wirksamkeit aufweisen, ohne die Haare zu beschweren.

Es wurde gefunden, dass sich für diesen Zweck insbesondere Mischungen verzweigter Ester ii) eignen.

In einer weiteren bevorzugten Ausführungsform enthalten erfindungsgemäße Haarbehandlungsmittel daher mindestens zwei unterschiedliche verzweigte Ester ii), wobei die Verzweigung im Alkoholrest oder im Säurerest sein kann.

Bevorzugte verzweigte Ester ii) können ausgewählt sein aus Verbindungen der Formel (I)

CH₃-(CH₂)ₘ-(CHR¹)ₙ-COO-R² (I),

in der
- R¹ für eine geradkettige oder verzweigte, gesättigte oder ungesättigte C₁-C₈-Alkylgruppe,
- m für eine Zahl von 3 bis 11,
- n für eine Zahl von 0 bis 3 und
- R² für eine geradkettige oder verzweigte, gesättigte oder ungesättigte C₁-C₂₄-Alkylgruppe stehen kann, die in der Kette eine oder mehrere Gruppierungen -OC(O)-, -NHC(O)- oder - C(OH)- enthalten kann.

In mehr bevorzugten Estern ii) der zuvor genannten Formel (I) steht
- R¹ für einen geradkettigen C₁-C₄-Alkylrest, ganz besonders bevorzugt für einen Methyl- oder einen Ethylrest,
- m für die Zahlen 3 bis 6,
- n für die Zahlen 0 oder 1 und
- R² für eine geradkettige, gesättigte C₁-C₁₈-Alkylgruppe oder für eine verzweigte, gesättigte C₁-C₁₈-Alkylgruppe, die in der Kette eine oder mehrere Gruppierungen -OC(O)- enthält.

Besonders bevorzugt enthalten die erfindungsgemäßen Haarbehandlungsmittel Mischungen aus mindestens einem Ester ii) der Formel (la) und mindestens einem Ester ii) (Ib)

CH₃-(CH₂)ₘ-(CHR¹)ₙ-COO-(CH₂)ₒ-CH₃ (Ia)

CH₃-(CH₂)ₘ-COO-(CH₂)ₚ-(CR³R⁴)_{q}-(CH₂)ᵣ-C(O)-O(CH₂)ₘ-CH₃ (Ib),

worin
- m, n und R¹ dieselbe Bedeutung haben wie oben, vorzugsweise in der m für die Zahlen 3, 4 oder 5; n für die Zahl 1 und R¹ für einen Methyl- oder Ethylrest steht,
- o für eine Zahl von 7 bis 19, vorzugsweise von 9 bis 17 steht,
- p und r unabhängig voneinander für die Zahlen 0, 1, 2 oder 3, vorzugsweise für die Zahl 1 oder 2 stehen,
- q für die Zahlen 0, 1, 2 oder 3, vorzugsweise für die Zahl 1 steht, und
- R³, R⁴ unabhängig voneinander für einen C₁-C₄-Alkylrest, vorzugsweise für einen Methylrest stehen.

Ganz besonders bevorzugte Ester nach Formel (la) sind Ester vernetzter C₅-C₈-Carbonsäuren und C₁₂-C₂₄-Monoalkoholen, vorzugsweise Ester von 2-Ethylhexylcarbonsäure und Myristyl-, Cetyl- und/oder Stearylalkohol und insbesondere unter der INCI-Bezeichnung Cetyl Ethylhexanoate bekannte Ester.

Ganz besonders bevorzugte Ester nach Formel (Ib) sind Neopentyl Polyol Ester, vorzugsweise Neopentyl Glycol Ester und insbesondere unter der INCI-Bezeichnung Neopentyl Glycol Diheptanoate bekannte Ester.

Ester ii), bevorzugt Ester nach Formel (Ib), mehr bevorzugt Neopentyl Polyol Ester, besonders bevorzugt Neopentyl Glycol Ester und insbesondere Neopentyl Glycol Diheptanoate können der Ölphase (II) der erfindungsgemäßen Haarbehandlungsmittel einzeln oder als Mischung mit einer oder mehreren Ölkomponenten (la) oder iii) hinzugefügt werden.

Weiterhin ist es möglich, Ester nach Formel (Ib), bevorzugt Neopentyl Polyol Ester, besonders bevorzugt Neopentyl Glycol Ester und insbesondere Neopentyl Glycol Diheptanoate als Mischung mit dem Isoparaffin i) in der Ölphase (II) der erfindungsgemäßen Haarbehandlungsmittel einzusetzen.

Solche Mischungen sind bekannt und werden als Handelsprodukte angeboten, beispielsweise unter der Bezeichnung Lexfeel^{®}D5 von der Firma Inolex.

In einer weiterhin bevorzugten Ausführungsform enthalten erfindungsgemäße Haarbehandlungsmittel in der Ölphase (II) als Ester ii)
- mindestens einen Neopentyl Polyol Polyester, vorzugsweise einen Neopentyl Glycol Diester und
- mindestens einen Ester einer vernetzten C₅-C₈-Carbonsäure und einem C₁₂-C₂₄-Monoalkohol, vorzugsweise Ester von 2-Ethylhexylcarbonsäure und Myristyl-, Cetyl- und/oder Stearylalkohol.

Innerhalb dieser Ausführungsform sind erfindungsgemäße Haarbehandlungsmittel besonders bevorzugt, die in der Ölphase (II) als Ester ii)
- mindestens einen unter der INCI-Bezeichnung Cetyl Ethylhexanoate bekannten Ester und
- mindestens einen unter der INCI-Bezeichnung Neopentyl Glycol Diheptanoate bekannten Ester enthalten.

Die Ester ii), vorzugsweise verzweigte Ester ii) gemäß Formel (I) und insbesondere eine Mischung aus Estern (la) und (Ib) können in den Ölphase (II) der erfindungsgemäßen Haarbehandlungsmittel bevorzugt in einem Gewichtsanteil von jeweils 48 bis 63 Gew.-%, besonders bevorzugt 50 bis 61 Gew.-% und insbesondere 52 bis 60 Gew.-% am Gesamtgewicht der Ölphase (II) eingesetzt werden.

Von besonderem Vorteil sind Estermischungen ii), vorzugsweise Mischungen von mindestens einem Ester der Formel (la) und mindestens einem Ester der Formel (Ib), in denen das Gewichtsverhältnis von Estern der Formel (la) zu Estern der Formel (Ib) im Bereich von 1 : 10 bis 1 : 2, vorzugsweise von 1 : 8 bis 1 : 3 und insbesondere von 1 : 7 bis 1 : 5 liegt.

Neben den beiden unterschiedlichen Estern ii) können die erfindungsgemäßen Haarbehandlungsmittel fakultativ weitere Ester enthalten, solange diese die Stabilität und die Pflegeeigenschaften der Mittel nicht negativ beeinträchtigen.

Als geeignete weitere Ester haben sich beispielsweise symmetrische, unsymmetrische oder cyclische Ester der Kohlensäure mit Fettalkoholen wie beispielsweise Dicaprylylcarbonat herausgestellt.

Dicaprylylcarbonat ist im Handel von mehreren Anbietern erhältlich, u.a. von der Firma BASF unter der Bezeichnung Cetiol^{®}.

Ester der Kohlensäure mit Fettalkoholen können in den erfindungsgemäßen Zusammensetzungen in etwa denselben Mengen eingesetzt werden wie die Ester gemäß Formel (la).

Als dritte wesentliche Komponente in der Ölphase (II) enthalten die erfindungsgemäßen Haarbehandlungsmittel mindestens ein pflanzliches Öl. Dadurch können erfindungsgemäße Zusammensetzung mit Inhaltsstoffen natürlichen Ursprungs angereichert werden, welche die Haare pflegen, ohne sie zu beschweren.

Unter geeigneten pflanzlichen Ölen iii) werden im Rahmen der vorliegenden Erfindung Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Hagebuttenkernöl, Haselnussöl, Holundersamenöl, Johannisbeersamenöl, Jojobaöl, Kakaobutter, Leinöl, Macadamianussöl, Maiskeimöl, Mandelöl, Marulaöl, Nachtkerzenöl, Olivenöl, Palmöl, Pfirsichkernöl, Rapsöl, Reisöl, Sanddornfruchtfleischöl, Sanddornkernöl, Sesamöl, Sheabutter, Sojaöl, Sonnenblumenöl, Traubenkernöl, Walnußöl oder Wildrosenöl verstanden.

Besonders geeignet sind zuvor genannte pflanzliche Öle, die wie die Öle i) und ii) ein sehr gute Verteilbarkeit auf der Kopfhaut und den Haaren aufweisen sowie schnell und gut in die Haarfasern einziehen und diese pflegen. Zu den ganz besonders geeigneten pflanzlichen Ölen zählen solche, die ähnlich den Ölen aus tierischen Quellen einen besonders hohen Anteil von Triglyceriden aus ungesättigten Fettsäuren wie Ölsäure und insbesondere Palmitoleinsäure enthalten. Beispiele für ganz besonders geeignete pflanzliche Öle im Sinne der vorliegenden Erfindung sind Avocadoöl, Sanddornfruchtfleischöl und/oder Macadamianussöl. Insbesondere bevorzugt ist Macadamianussöl.

Das mindestens eine pflanzliche Öl iii), vorzugsweise Avocadoöl, Sanddornfruchtfleischöl und/oder Macadamianussöl und insbesondere Macadamisanussöl kann in den Ölphase (II) der erfindungsgemäßen Haarbehandlungsmittel bevorzugt in einem Gewichtsanteil von jeweils 6 bis 17 Gew.-%, besonders bevorzugt 7 bis 15 Gew.-% und insbesondere 8 bis 12 Gew.-% am Gesamtgewicht der Ölphase (II) eingesetzt werden.

In einer ganz besonders bevorzugten Ausführungsform enthalten erfindungsgemäße Haarbehandlungsmittel in der Ölphase (II) - bezogen auf das Gesamtgewicht der Ölphase -
- 20 bis 40 Gew.-%, mehr bevorzugt 22 bis 38 Gew.-%, besonders bevorzugt 25 bis 35 Gew.-% und insbesondere 27 bis 33 Gew.-% Isoundecan, Isododecan und/oder Isotridecan,
- 10 bis 60 Gew.-%, mehr bevorzugt 15 bis 57,5 Gew.-%, besonders bevorzugt 20 bis 55 Gew.-% und insbesondere 25 bis 52,5 Gew.-% mindestens eines Neopentyl Glycol Diesters, insbesondere unter der INCI-Bezeichnung Neopentyl Glycol Diheptanoate bekannte Verbindungen,
- 5 bis 20 Gew.-%, mehr bevorzugt 6 bis 17 Gew.-%, besonders bevorzugt 7 bis 15 Gew.-% und insbesondere 8 bis 12 Gew.-% Ester von 2-Ethylhexylcarbonsäure und Myristyl-, Cetyl- und/oder Stearylalkohol. insbesondere unter der INCI-Bezeichnung Cetyl Ethylhexanoate bekannte Verbindungen, und
- 5 bis 20 Gew.-%, mehr bevorzugt 6 bis 17 Gew.-%, besonders bevorzugt 7 bis 15 Gew.-% und insbesondere 8 bis 12 Gew.-% Avocadoöl, Sanddornfruchtfleischöl und/oder Macadamianussöl, insbesondere Macadamisanussöl.

Für die Optimierung der Pflegeeigenschaften der erfindungsgemäßen Haarbehandlungsmittel ist es vorteilhaft, wenn der Wasserphase (I) ebenfalls Haarpflegewirkstoffe zugesetzt werden. Bei der Wahl dieser Pflegewirkstoffe ist zu beachten, dass sie weder die Stabilität der Wasserphase (I) noch die Stabilität des Zweiphasenproduktes negativ beeinträchtigen.

Optimalerweise werden in der Wasserphase (I) daher nur Pflegewirkstoffe eingesetzt, die in der Wasserphase (I) gut löslich sind und/oder die die Viskosität der Wasserphase nicht auf Werte außerhalb der zuvor genannten Grenzen erhöhen.

Beispiele für geeignete (Pflege)-Wirkstoffe, die der Wasserphase (I) vorteilhafterweise hinzugefügt werden, können ausgewählt sein aus
- kationischen Tensiden und/oder
- kationischen Polymeren und/oder
- Proteinhydrolysaten und/oder
- Vitaminen und/oder
- Wirkstoffen zur pH-Regulierung.

Weiterhin kann die Wasserphase Konservierungsmittel, beispielsweise Benzoesäure und/oder ein physiologisch verträgliches Salz der Benzoesäure enthalten.

In einer weiteren bevorzugten Ausführungsform enthalten die erfindungsgemäßen Haarbehandlungsmittel in der Wasserphase (I) - bezogen auf das Gesamtgewicht der Wasserphase (I) - 0,01 bis 1,0 Gew.-% mindestens eines kationischen Tensids.

Besonders bevorzugt werden die kationischen Tenside in Mengen von 0,05 bis 0,8 Gew.-%, ganz besonders bevorzugt von 0,075 bis 0,6 Gew.-% und insbesondere von 0,10 bis 0,5 Gew.-% (bezogen auf das Gesamtgewicht der Wasserphase (I)) eingesetzt, denn sie sollen den Haarpflegeeffekt verstärken, nicht aber zu einer Verlängerung der Emulgierung der Ölphase (II) (oder zu Teilen der Ölphase) beitragen.

Unter geeigneten kationischen Tensiden für die Verwendung in den erfindungsgemäßen Haarbehandlungsmitteln werden vorzugsweise lineare oder verzweigte, gesättigte oder ungesättigte physiologisch verträgliche C₁-C₂₄-Alkyltri(C₁-C₄)-alkylammoniumsalze, Di-C₁-C₂₄-Alkyldi(C₁-C₄)-alkylammoniumsalze oder Tri-C₁-C₂₄-Alkyl(C₁-C₄)-alkylammoniumsalze und/oder primäre, sekundäre oder tertiäre Amine, die mindestens eine Gruppe R-CO(NH)-(CH₂)ₙ- enthalten, in der R für eine lineare oder verzweigte, gesättigte oder ungesättigte Alkylgruppe mit 7 bis 21 C-Atomen und n für eine ganze Zahl von 1 bis 4 steht, verstanden.

Besonders bevorzugte C₁-C₂₄-Alkyltri(C₁-C₄)-alkylammoniumsalze, Di-C₁-C₂₄-Alkyldi(C₁-C₄)-alkylammoniumsalze oder Tri-C₁-C₂₄-Alkyl(C₁-C₄)-alkylammoniumsalze sind beispielsweise Halogenid- und/oder Methosulfatsalze, insbesondere Chloride, Bromide und Methosulfate, wie beispielsweise Distearyldimethylammoniumchlorid, Lauryltrimethylammoniumchlorid, Lauryltrimethylbenzylammoniumchlorid, Tricetylmethylammoniumchlorid, Cetyltrimethylammoniumchlorid, Cetyltrimethylammoniumbromid, Cetyltrimethylammoniummethosulfat, Stearyltrimethylammoniumchlorid, Behenyltrimethylammoniumchlorid, Behenyltrimethylammoniumbromid und Behenyltrimethylammoniummethosulfat.

Ganz besonders bevorzugt sind Cetyltrimethylammonium- und Behenyltrimethylammoniumsalze und insbesondere Cetyltrimethylammoniumsalze, die als Anion ein Methosulfat- und/oder ein Chloridion enthalten.

Besonders bevorzugte primäre, sekundäre oder tertiäre Amine, die mindestens eine Gruppe R-CO(NH)-(CH₂)ₙ- enthalten, in der R für eine lineare oder verzweigte, gesättigte oder ungesättigte Alkylgruppe mit 7 bis 21 C-Atomen, und n für eine ganze Zahl von 1 bis 4 steht, sind bekannt unter der Bezeichnung "Amidoamin".

Geeignete Amidoamine können sowohl als solche, als auch (infolge einer Protonierung in entsprechend saurer Lösung) jeweils in der Form ihrer entsprechenden quaternären Verbindung in den Haarkonditioniermitteln vorliegen.

Bevorzugt sind die nicht-kationischen Amidoamine.

Besonders geeignete Amidoamine, welche gegebenenfalls quaternisiert sein können, sind beispielsweise Tego Amid^{®} S18 (Evonik; INCI-Bezeichnung: Stearamidopropyl Dimethylamine), Lexamine^{®} S13 (Inolex; INCI-Bezeichnung: Stearamidopropyl Dimethylamine), Incromine^{®} SB (Croda; INCI-Bezeichnung: Stearamidopropyl Dimethylamine), Witcamine^{®} 100 (Witco, INCI-Bezeichnung: Cocamidopropyl Dimethylamine), Incromine^{®} BB (Croda, INCI-Bezeichnung: Behenamidopropyl Dimethylamine), ProCondition^{®}22 (Inolex, INCI-Bezeichnung: Brassicamidopropyl Dimethylamine), Mackine^{®} 401 (Mclntyre, INCI-Bezeichnung: Isostearylamidopropyl Dimethylamine) und andere Mackine-Typen sowie Adogen^{®} S18V (Witco, INCI-Bezeichnung: Stearylamidopropyl Dimethylamine).

Als permanent kationische Amidoamine können beispielsweise eingesetzt werden: Rewoquat^{®} RTM 50 (Witco Surfactants GmbH, INCI-Bezeichnung: Ricinoleamidopropyltrimonium Methosulfate), Empigen^{®} CSC (Albright & Wilson, INCI-Bezeichnung: Cocamidopropyltrimonium Chlorid), Swanol Lanoquat^{®} DES-50 (Nikko, INCI-Bezeichnung: Quatemium-33), Rewoquat^{®} UTM 50 (Witco Surfactants GmbH, Undecyleneamidopropyltrimonium Methosulfate).

Insbesondere bevorzugt sind Stearamidopropyl Dimethylamine und/oder Brassicamidopropyl Dimethylamine.

In einer weiteren bevorzugten Ausführungsform enthalten die erfindungsgemäßen Haarbehandlungsmittel in der Wasserphase (I) - bezogen auf das Gesamtgewicht der Wasserphase (I) - 0,01 bis 2,5 Gew.-% mindestens eines kationischen Polymeren.

Unter geeigneten kationischen Polymeren für die Verwendung in den erfindungsgemäßen Haarbehandlungsmitteln werden vorzugsweise kationische Polymere verstanden, die bekannt sind unter der INCI-Bezeichnung "Polyquaternium".

Besonders geeignete kationische Polymere sind Polyquaternium-6, Polyquaternium-7, Polyquaternium-10, Polyquaternium-11, Polyquaternium-16, Polyquaternium-22, Polyquaternium-24, Polyquaternium-28, Polyquaternium-37, Polyquaternium-67, Polyquaternium-74 und/oder Polyquaternium-89.

Insbesondere geeignet sind kationische Polymere, die sich von Polymeren natürlichen Ursprungs wie Cellulose-, Stärke- oder Guarpolymeren ableiten.

Kationische Cellulosederivate werden beispielsweise von der Firma Amerchol unter der Bezeichnung Polymer JR^{®} und kationische Guarpolymere von der Firma Rhöne-Poulenc oder Hercules unter den Bezeichnungen Jaguar^{®} oder N-Hance^{®} angeboten.

Weitere geeignete kationische Polymere natürlichen Ursprungs sind beispielsweise kationische Chitosane, die beispielsweise unter den Bezeichnungen "Kytamer" oder "Hydagen" im Handel erhältlich sind.

Weiterhin geeignet sind die unter der INCI-Bezeichnung "Quaternium" bekannten kationischen Polymere wie Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 und/oder Quaternium-84.

Weitere geeignete kationische Polymere sind beispielsweise - kationische Alkylpolyglycoside,-kationisierter Honig, beispielsweise das Handelsprodukt Honeyquat^{®} 50,- Vinylpyrrolidon-Vinylimidazoliummethochlorid-Copolymere, wie sie unter den Bezeichnungen Luviquat^{®} FC 370, FC 550, FC 905 und HM 552 angeboten werden,- quaternierter Polyvinylalkohol,- sowie die unter den Bezeichnungen Polyquaternium-2, Polyquaternium-17, Polyquaternium-18 und Polyquaternium-27 bekannten Polymere mit quartären Stickstoffatomen in der Polymerhauptkette,-Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Besonders bevorzugt werden in den erfindungsgemäßen Haarbehandlungsmitteln kationische Polymere eingesetzt, die aus Guar Hydroxypropyltrimonium Chloride, Polyquaternium-10, Polyquaternium-11, Polyquaternium-16, Polyquaternium-37, Polyquaternium-67, Polyquaternium-74 und/oder Polyquaternium-89 ausgewählt sind. Insbesondere bevorzugt ist Polyquaternium-16.

In einer weiteren bevorzugten Ausführungsform enthalten die erfindungsgemäßen Haarbehandlungsmittel in der Wasserphase (I) - bezogen auf das Gesamtgewicht der Wasserphase (I) - 0,01 bis 1,0 Gew.-% mindestens eines kationischen Tensids und 0,01 bis 2,5 Gew.-% mindestens eines kationischen Polymeren.

Unter geeigneten Proteinhydrolysaten (PH) für die Verwendung in den erfindungsgemäßen Haarbehandlungsmitteln werden sowohl PH pflanzlichen als auch tierischen, marinen oder synthetischen Ursprungs verstanden.

Bevorzugte tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Bevorzugte pflanzliche Proteinhydrolysate sind beispielsweise Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Zu den geeigneten Proteinhydrolysaten maritimen Ursprunges zählen beispielsweise Kollagenhydrolysate von Fischen oder Algen sowie Proteinhydrolysate von Muscheln bzw. Perlenhydrolysate. Beispiele für erfindungsgemäß geeignete Perlenhydrolysate sind die Handelsprodukte Pearl Protein Extract BG^{®} oder Crodarom^{®} Pearl.

Geeignete kationisierte Proteinhydrolysate können ebenfalls tierischen, pflanzlichen oder maritimen Ursprungs sein.

Die Quaternisierung der Proteinhydrolysate oder der Aminosäuren kann mit quarternären Ammoniumsalzen wie beispielsweise N,N-Dimethyl-N-(n-Alkyl)-N-(2-hydroxy-3-chloro-n-propyl)-ammoniumhalogeniden durchgeführt werden. Darüber hinaus können die kationischen Proteinhydrolysate auch noch weiter derivatisiert sein.

Als typische Beispiele für besonders geeignete kationische Proteinhydrolysate und/oder deren Derivate seien die unter den INCI-Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Besonders bevorzugt für die Verwendung in den erfindungsgemäßen Haarbehandlungsmitteln sind Keratin-, Seiden-, Milcheiweiß-, Weizen- und/oder Sojaproteinhydrolysate, die quaternisiert sein können. Insbesondere bevorzugt sind Keratin- und/oder Weizen- und/oder Seidenproteinhydrolysate, die quaternisiert sein können.

Die - gegebenenfalls quaternisierten - Proteinhydrolysate können in den erfindungsgemäßen Haarbehandlungsmitteln sowohl einzeln als auch als Mischung eingesetzt werden.

Es kann für einige Ausführungsformen der Erfindung von Vorteil sein, wenn die Haarbehandlungsmittel mindestens ein Proteinhydrolysat tierischen oder pflanzlichen Ursprungs sowie mindestens ein quaternisiertes Proteinhydrolysat tierischen oder pflanzlichen Ursprungs enthalten.

Die Proteinhydrolysate und/oder quaternisierten Proteinhydrolysate können in der Wasserphase (I) der erfindungsgemäßen Haarbehandlungsmittel - bezogen auf das Gesamtgewicht der Wasserphase (I) - bevorzugt in Mengen von 0,001 bis 5 Gew.-%, vorzugsweise von 0,0025 bis 2,5 Gew.-% und insbesondere in Mengen von 0,005 bis 1 Gew.-% enthalten sein.

Unter geeigneten Vitaminen, die für die Verwendung in den erfindungsgemäßen Haarbehandlungsmitteln geeignet sind, werden Vitamine, Provitamine und Vitaminvorstufen sowie deren Derivate aus den Gruppen A, B, C, E, F und H verstanden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht. Die Haarbehandlungsmittel können die Vitamin A-Komponente bevorzugt in Mengen von 0,005-1 Gew.-%, bezogen auf das gesamte Mittel, enthalten.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Bevorzugt ist das Nicotinsäureamid, das in den Haarbehandlungsmitteln bevorzugt in Mengen von 0,005 bis 1 Gew.-%, bezogen auf das gesamte Mittel, enthalten sein kann
- Vitamin B₅ (Pantothensäure, Panthenol und Pantolacton).
   Im Rahmen dieser Gruppe wird bevorzugt das Panthenol und/oder Pantolacton eingesetzt. Einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Die genannten Verbindungen des Vitamin B₅-Typs können in den Haarbehandlungsmitteln bevorzugt in Mengen von 0,05-5 Gew.-%, bezogen auf das gesamte Mittel, enthalten sein. Mengen von 0,1-2 Gew.-% sind besonders bevorzugt
- Vitamin B₆ (Pyridoxin sowie Pyridoxamin und Pyridoxal)
- Vitamin B₇ - vgl. Vitamin H.

Vitamin C (Ascorbinsäure). Vitamin C kann in den Haarbehandlungsmitteln bevorzugt in Mengen von 0,01 bis 3 Gew.-%, bezogen auf das gesamte Mittel, eingesetzt werden. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, können in den Haarbehandlungsmitteln bevorzugt in Mengen von 0,005-1 Gew.-%, bezogen auf das gesamte Mittel, enthalten sein.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S,6aR)-2-Oxohexahydrothienol[3,4-d]imidazol-4-valeriansäure bezeichnet, für die sich aber inzwischen der Trivialname Biotin durchgesetzt hat. Biotin kann in den Haarbehandlungsmitteln bevorzugt in Mengen von 0,0001 bis 1,0 Gew.-%, insbesondere in Mengen von 0,001 bis 0,01 Gew.-% enthalten sein.

Besonders bevorzugt können die erfindungsgemäßen Haarbehandlungsmittel Vitamine, Provitamine und Vitaminvorstufen aus den Gruppen A, B, E und H enthalten. Panthenol, Pantolacton, Pyridoxin und seine Derivate sowie Nicotinsäureamid und Biotin sind besonders bevorzugt und können den erfindungsgemäßen Haarkonditioniermitteln sowohl einzeln, als auch in ihrer Kombination in den zuvor genannten Mengen zugesetzt werden.

Unter geeigneten Wirkstoffen zur pH-Regulierung, die für die Verwendung in den erfindungsgemäßen Haarbehandlungsmitteln geeignet sind, werden vorzugsweise Säuren wie insbesondere Essigsäure, Citronensäure, Äpfelsäure, Maleinsäure, Ameisensäure, Amidosulfonsäure, Phosphorsäure, Phosphonsäuren, D-Milchsäure, L-Milchsäure, D/L-Milchsäure, Oxalsäure oder aus der Gruppe der Alkalien, wie insbesondere Natronlauge, vorteilhafterweise in Mengen von 0,01 - 10 Gew.-%, bevorzugt 0,01 - 5 Gew.-% und insbesondere von 0,01 bis 2 Gew.-% (bezogen auf das Gesamtgewicht des Haarbehandlungsmittels) verstanden.

Bevorzugt werden Wirkstoffe zur pH-Regulierung in den erfindungsgemäßen Haarbehandlungsmitteln eingesetzt, die neben ihrer Funktion zur pH-Wert-Einstellung weiterhin positive Eigenschaften auf den Haaren und/oder der Kopfhaut gewährleisten können. In diesem Zusammenhang ist insbesondere Milchsäure zu nennen, denn es kann auf der Kopfhaut zur Unterstützung der Hautfeuchtigkeitsregulierung dienen und auf ggfs. gefärbten Haaren zum Farbschutz beitragen.

Es wurde gefunden, dass es für eine besonders gute Stabilität der beiden Phasen (I) und (II) des erfindungsgemäßen Haarbehandlungsmittels von Vorteil ist, wenn sie nur bestimmte Tenside und/oder Emulgatoren in bestimmten Mengen enthalten.

Unter "Stabilität" wird in diesem Zusammenhang verstanden, dass die beiden Phasen nach der Durchmischung schnell wieder separieren und einen scharfe horizontaler Phasengrenze bilden, an der über einen langen Zeitraum und bei Temperaturschwankungen optimalerweise keine Trübungen auftreten.

Tenside und/oder Emulgatoren, welche die Emulgierung und/oder Suspendierung des Ölphase in der Wasserphase oder anders herum zu stark begünstigen sind demzufolge für die Verwendung in den erfindungsgemäßen Zusammensetzungen nicht geeignet.

Weiterhin unerwünscht ist eine Schaumbildung beim Schütteln des erfindungsgemäßen Haarbehandlungsmittels unmittelbar vor der Anwendung, weshalb auch Tenside / Emulgatoren ausgeschlossen sind, die zu starker Schaumbildung neigen.

In einer bevorzugten Ausführungsform enthalten die erfindungsgemäßen Haarbehandlungsmittel maximal 1 Gew.-%, mehr bevorzugt maximal 0,8 Gew.-%, besonders bevorzugt maximal 0,6 Gew.-% und insbesondere maximal 0,5 Gew.-% anionische, amphotere, zwitterionische und/oder nichtionische Tenside und/oder Emulgatoren (bezogen auf das Gesamtgewicht des Haarbehandlungsmittels).

Innerhalb dieser Ausführungsform ist es besonders bevorzugt, wenn die erfindungsgemäßen Haarbehandlungsmittel keine weiteren anionischen, amphoteren, zwitterionischen und/oder nichtionischen Tenside und/oder Emulgatoren enthalten.

Weiterhin wurde beobachtet, dass die Anwesenheit diverser Öl- und Fettstoffe die Entmischung der beiden Phasen (I) und (II) nach deren Homogenisierung erschweren kann.

Von besonderem Vorteil sind daher erfindungsgemäße Haarbehandlungsmittel, die in ihrer Ölphase (II) neben den bereits beschriebenen wesentlichen und fakultativen Inhaltsstoffen maximal 0,5 Gew.-%, besonders bevorzugt maximal 0,3 Gew.-% und insbesondere maximal 0,1 Gew.-% weiterer Öl- und/oder Fettstoffe enthalten (bezogen auf die Ölphase (II)).

Optimalerweise enthalten die erfindungsgemäßen Haarbehandlungsmittel neben den an früherer Stelle dieser Anmeldung beschriebenen Bestandteilen der Ölphase (II) keine weiteren Öl- und/oder Fettstoffe, insbesondere keine Fettalkohole.

In einer weiteren bevorzugten Ausführungsform sind erfindungsgemäße Haarbehandlungsmittel im Wesentlichen frei von Fettalkoholen.

Unter "im Wesentlichen frei" wird verstanden, dass die erfindungsgemäßen Haarbehandlungsmittel - bezogen auf ihr Gesamtgewicht - maximal 0,05 Gew.-%, besonders bevorzugt maximal 0,01 Gew.-% und insbesondere 0 Gew.-% Fettalkohole enthalten.

Nicht zu den zuvor genannten Öl- und Fettstoffen zu rechnen sind in diesem Zusammenhang Parfumöle. Diese können in der Ölphase bevorzugt in einer Menge von bis zu 5 Gew.-%, mehr bevorzugt 4 Gew.-%, besonders bevorzugt 3 Gew.-% und insbesondere 2 Gew.-% (bezogen auf das Gesamtgewicht der Ölphase) eingesetzt werden.

Ferner können die erfindungsgemäßen Haarbehandlungsmittel weitere Wirk-, Hilfs- und Zusatzstoffe enthalten, wie beispielsweise
- Strukturanten wie Maleinsäure
- Farbstoffe zum Anfärben des Mittels
- Antischuppenwirkstoffe wie Piroctone Ölamine, Zink Omadine und Climbazol
- Cholesterin
- Komplexbildner wie EDTA, NTA, β-Alanindiessigsäure und Phosphonsäuren
- Pigmente
- Antioxidantien und/oder UV-Filter
- amphotere, anionische und/oder anionische Polymere
- Pflanzenextrakte
- Penetrationshilfsmittel und/oder Quellmittel wie Harnstoff, Hydantoin und/oder deren Derivate.

Die erfindungsgemäßen Haarkonditioniermittel weisen bevorzugt einen pH-Wert im Bereich von 2 bis 7, bevorzugt von 2,5 bis 6 und insbesondere von 3 bis 4 auf.

Neben einem ästhetischen Erscheinungsbild haben die erfindungsgemäßen zweiphasigen Haarbehandlungsmittel den Vorteil, dass sie leicht in der Handhabung und einfach in ihrer Herstellung sind. Die spezielle Wirkstoffkombination in den Haarbehandlungsmitteln gewährleistet eine schnelle Durchmischung der beiden Phasen vor der Anwendung und eine schnelle Entmischung der beiden Phasen nach der Anwendung. Die hydrophile Phase basiert überwiegend auf Wasser, was den Bedürfnissen der Verbraucher entgegenkommt. Die erfindungsgemäßen Haarbehandlungsmittel eignen sich darüber hinaus besonders für die Pflege von Haaren. Die Anwesenheit (cyklischer) Silikone ist weder für die Stabilität noch für die Pflegeeigenschaften der Haarbehandlungsmittel erforderlich, denn die erfindungsgemäßen Haarbehandlungsmittel weisen auf damit behandelten Haaren gegenüber Silikone-enthaltenden Mitteln vergleichbar gute Konditionierungsvorteile auf. Insbesondere der Haarglanz und die Frisier- und Stylingeigenschaften insbesondere vorher strapazierter und/oder geschädigter Haare konnten signifikant verbessert werden. Doch auch die "anti-frizz"-Wirkung derartiger Haarbehandlungsmittel wurde in einem Verbrauchertest hervorragend bewertet. Die erfindungsgemäßen Haarbehandlungsmittel sind versprühbar und eignen sich für die Applikation aus einem Pumpspender. Dadurch sind sie einfach in der Handhabung und effektiv in ihrer Wirkung, denn der aus dem Pumpspender austretende feine Sprühnebel erreicht alle Haarbereiche.

Ein zweiter Gegenstand der Erfindung ist ein Verfahren zur Konditionierung von Haaren, bei dem ein erfindungsgemäßes Haarbehandlungsmittel nach kräftigem Schütteln auf die nassen oder trockenen Haare aufgebracht (vorzugsweise gesprüht) und bis zur nächsten Haarreinigung auf den Haaren belassen wird.

Ein dritter Gegenstand der Erfindung ist die Verwendung des erfindungsgemäßen Haarbehandlungsmittels zur Verbesserung
- der Nass- und/oder Trockenkämmbarkeit
- des Haargefühls bis in die Haarspitzen (in nassen und trockenen Haaren)
- der Griffigkeit der Haare
- der Frisierbarkeit / Formgebung der Frisur
- der Anti-Frizz-Eigenschaften
- der Geschmeidigkeit von Haaren
- des Haarglanzes und/oder
- der statischen Aufladung von Haaren.

Für das erfindungsgemäße Verfahren und die erfindungsgemäße Verwendung gilt das zu den erfindungsgemäßen Mitteln Gesagte.

Die folgenden Beispiele sollen die Gegenstände der Erfindung näher beschreiben, ohne sie darauf zu beschränken.

Es wurden die erfindungsgemäßen Zusammensetzungen (I) und (II) sowie eine Vergleichszusammensetzung (III) hergestellt.
erfindungsgemäße Zusammensetzung (I) wurde mit der Silikone-enthaltenden Zusammensetzung aus dem Stand der Technik (III) verglichen. Die Mengenangaben beziehen sich auf Gew.-% (in der gesamten Zusammensetzung):

| | **(I)** | **(II)** | **(III)** |
|---|---|---|---|
| **Ölphase** | | | |
| LexFeel^{®1} D5 | 9,00 | 9,00 | |
| Schercemol^{®2} CO Ester | 1,00 | 1,00 | |
| Cetil^{®3} CC | | 1,00 | |
| Macadamianussöl | 1,00 | 1,00 | |
| Parfum | 0,25 | 0,25 | 0,25 |
| Phenyl Trimethicone | | | 1,00 |
| DC 1501^{®4} | | | 4,50 |
| Cyclopentasiloxan | | | 3,00 |
| Microcare Silicone^{®5} E1016 | | | 0,50 |
| | | | |

| **Wasserphase** | | | |
|---|---|---|---|
| Cetrimonium Chloride | 0,15 | 0,15 | 0,15 |
| Polyquaternium-16 | 0,40 | 0,40 | 0,40 |
| Panthenol | 0,20 | 0,20 | 0,20 |
| Wasser, Wirkstoffe zur pH-Regulierung (pH 4), Konservierungsmittel, Farbstoffe | ad 100 | ad 100 | ad 100 |

| | |
|---|---|
| ^{®1} | INCI-Bezeichnung: Neopentyl Glycol Diheptanoate, Isododecane (Inolex) |
| ^{®2} | INCI-Bezeichnung: Cetyl Erhylhexanoate (Lubrizol) |
| ^{®3} | INCI-Bezeichnung: Dicaprylyl Carbonate (BASF) |
| ^{®4} | INCI-Bezeichnung: Cyclomethicone, Dimethiconol (Dow Corning) |
| ^{®5} | INCI-Bezeichnung: Cetyl PEG/PPG-10/1 Dimethicone (Thor) |

In einem Test wurden die beiden Zusammensetzungen von Probanden hinsichtlich einer Reihe von Produkt - und Pflegeeigenschaften bewertet (Konsistenz des Produktes; Verteilbarkeit; Geruch; Erwirrbarkeit nasser Haare; Kämmbarkeit nasser und trockener Haare; Haargefühl nasser und trockener Haare; Formbarkeit / Frisierbarkeit; Haargefühl der Haarspitzen; Statische Aufladung; Glanz; Fülle der Haare; Pflegegrad; Weichheit der Haare; Geschmeidigkeit der Haare; Moisture; Überkonditionierung; Builtup; Gesamtbewertung).

Die beiden Produkte wurden neutral verpackt und codiert, in gleichen Mengen an drei Modellen in einem Halbseitentest angewendet und von einem Experten beurteilt.

Die glatten, unbehandelten Haare der Modelle 1 und 2 wiesen einen normalen (gesunden) Haar- und Kopfhautzustand sowie eine mittlere Dichte und einen mittleren Haardurchmesser auf.

Die gebleichten Haare des Modells 3 waren porös, von geringerer Dichte und geringerem Haardurchmesser.

Es konnten die Noten 1 bis 6 (wobei 1 für schlecht und 6 für sehr gut stand) vergeben werden.

Die Auswertung ergab, dass die erfindungsgemäße Zusammensetzung (I) in fast allen zuvor genannten Kategorien in etwa gleich mit der Zusammensetzung (III) bewertet wurde. Insbesondere hinsichtlich Formgebung / Frisierbarkeit und Glanz sowie in der Gesamtbewertung wurde die erfindungsgemäße Zusammensetzung (I) besser bewertet als die Silikone-enthaltende Vergleichszusammensetzung (III).

## Patentansprüche

1. Ein Haarbehandlungsmittel in Form eines Zweiphasensystems mit zwei separaten, optisch erkennbaren kontinuierlichen Phasen mit gemeinsamer horizontaler Phasengrenze, enthaltend
- bezogen auf das Gesamtgewicht des Haarbehandlungsmittels - eine Wasserphase (I) in einem Gewichtsanteil von mindestens 85 Gew.-% und eine Ölphase (II) in einem Gewichtsanteil von höchstens 15 Gew.-%, wobei
- die Ölphase (II) - bezogen auf ihr Gesamtgewicht - eine Mischung aus
i) 20 bis 40 Gew.-% mindestens eines Isoparaffins mit 9 bis 17 Kohlenstoffatomen,
ii) 45 bis 65 Gew.-% mindestens zweier unterschiedlicher Mono- und/oder Diester von linearen oder verzweigten, gesättigten oder ungesättigten C₄-C₁₂-Carbonsäuren mit linearen oder verzweigten, gesättigten oder ungesättigten C₄-C₂₄-Mono- oder Dialkoholen, und
iii) 5 bis 20 Gew.-% mindestens eines pflanzlichen Öls enthält, und
- die Haarbehandlungsmittel frei von Silikonverbindungen sind.

2. Haarbehandlungsmittel nach Anspruch 1, enthaltend
a) die Wasserphase (I) in einem Gewichtsanteil von mindestens 88 Gew.-% am Gesamtgewicht des Haarbehandlungsmittels und
b) die Ölphase (II) in einem Gewichtsanteil von höchstens 12 Gew.-% am Gesamtgewicht des Haarbehandlungsmittels.

3. Haarbehandlungsmittel nach einem der vorhergehenden Ansprüche, enthaltend als Isoparaffin i) Isodecan, Isoundecan, Isododecan, Isotridecan, Isotetradecan sowie Mischungen davon.

4. Haarbehandlungsmittel nach Anspruch 3, enthaltend Isoundecan, Isododecan und/oder Isotridecan.

5. Haarbehandlungsmittel nach einem der vorhergehenden Ansprüche, enthaltend als mindestens zwei unterschiedliche Ester ii) verzweigte Ester, wobei die Verzweigung im Alkoholrest oder im Säurerest sein kann.

6. Haarbehandlungsmittel nach Anspruch 5, enthaltend
- mindestens einen Neopentyl Polyol Polyester, vorzugsweise einen Neopentyl Glycol Diester, und
- mindestens einen Ester einer vernetzten C₅-C₈-Carbonsäure und einem C₁₂-C₂₄-Monoalkohol, vorzugsweise Ester von 2-Ethylhexylcarbonsäure und Myristyl-, Cetyl- und/oder Stearylalkohol.

7. Haarbehandlungsmittel nach einem der vorhergehenden Ansprüche, enthaltend als pflanzliches Öl iii) Macadamianussöl.

8. Haarbehandlungsmittel nach einem der vorhergehenden Ansprüche, enthaltend in der Wasserphase (I), bezogen auf deren Gesamtgewicht, zusätzlich 0,01 bis 2,5 Gew.-% mindestens eines kationischen Polymeren.

9. Haarbehandlungsmittel nach einem der vorhergehenden Ansprüche, enthaltend in der Wasserphase (I), bezogen auf deren Gesamtgewicht, zusätzlich 0,01 bis 1,0 Gew.-% mindestens eines kationischen Tensids.

10. Haarbehandlungsmittel nach einem der vorhergehenden Ansprüche, enthaltend in der Wasserphase (I), bezogen auf deren Gesamtgewicht, > 85 Gew.-%, vorzugsweise > 90 Gew.-% und insbesondere > 95 Gew.-% Wasser.

11. Haarbehandlungsmittel nach einem der vorhergehenden Ansprüche, das frei von Fettalkoholen ist.

12. Haarbehandlungsmittel nach einem der vorhergehenden Ansprüche, wobei die Wasserphase (I) und die Ölphase (II) jeweils eine Viskosität < 1000mPas, vorzugsweise < 750mPas und besonders bevorzugt < 500mPas aufweisen (gemessen bei 20°C mit einem Brookfield-Viskosimeter DV-II, Spindel 2, 20 UpM).

13. Verfahren zur Konditionierung von Haaren, bei dem ein Haarbehandlungsmittel nach einem der Ansprüche 1 bis 12 nach kräftigem Schütteln auf die nassen oder trockenen Haare aufgebracht (vorzugsweise gesprüht) und bis zur nächsten Haarreinigung auf den Haaren belassen wird.

14. Kosmetische Verwendung eines Haarbehandlungsmittels nach einem der Ansprüche 1 bis 12 zur Verbesserung
- der Nass- und/oder Trockenkämmbarkeit
- des Haargefühls bis in die Haarspitzen (in nassen und trockenen Haaren)
- der Griffigkeit der Haare
- der Frisierbarkeit / Formgebung der Frisur
- der Anti-Frizz-Eigenschaften
- der Geschmeidigkeit von Haaren
- des Haarglanzes und/oder
- der statischen Aufladung von Haaren.

## Claims

1. A hair treatment agent in the form of a two-phase system comprising two separate, visually distinguishable continuous phases with a common horizontal phase boundary, containing-based on the total weight of the hair treatment agent-an aqueous phase (I) in a weight percentage of at least 85 wt.% and an oil phase (II) in a weight percentage of at most 15 wt.%, wherein
- the oil phase (II)-based on its total weight-comprises a mixture of
i) 20 to 40 wt.-% of at least one isoparaffin having 9 to 17 carbon atoms,
ii) 45 to 65 wt.-% of at least two different mono- and/or diesters of linear or branched, saturated or unsaturated C₄-C₁₂carboxylic acids with linear or branched, saturated or unsaturated C₄-C₂₄mono- or diols, and
iii) 5 to 20 wt.-% of at least one vegetable oil, and
- the hair treatment products are free of silicone compounds.

2. A hair treatment product according to claim 1, comprising
a) the aqueous phase (I) in a weight fraction of at least 88% by weight of the total weight of the hair treatment product, and
b) the oil phase (II) in a weight percentage of no more than 12% by weight of the total weight of the hair treatment product.

3. A hair treatment product according to any of the preceding claims, containing, as isoparaffins, i) isodecane, isoundecane, isododecane, isotridecane, isotetradecane, and mixtures thereof.

4. A hair treatment composition according to claim 3, comprising isoundecane, isododecane, and/or isotridecane.

5. A hair treatment composition according to any of the preceding claims, containing, as at least two different esters, ii) branched esters, wherein the branching may be in the alcohol moiety or in the acid moiety.

6. Hair treatment composition according to claim 5, comprising
- at least one neopentyl polyol polyester, preferably a neopentyl glycol diester, and
- at least one ester of a cross-linked C₅-C₈carboxylic acid and a C₁₂-C₂₄monohydroxy alcohol, preferably esters of 2-ethylhexylcarboxylic acid and myristyl, cetyl, and/or stearyl alcohol.

7. A hair treatment composition according to any of the preceding claims, comprising, as a vegetable oil, iii) macadamia nut oil.

8. A hair treatment composition according to any of the preceding claims, comprising, in the aqueous phase (I), based on its total weight, an additional 0.01 to 2.5 wt.-% of at least one cationic polymer.

9. A hair treatment composition according to any of the preceding claims, comprising, in the aqueous phase (I), based on its total weight, an additional 0.01 to 1.0% by weight of at least one cationic surfactant.

10. A hair treatment composition according to one of the preceding claims, comprising, in the aqueous phase (I), based on its total weight, > 85 wt.%, preferably > 90 wt.%, and in particular > 95 wt.% water.

11. A hair treatment composition according to any of the preceding claims, which is free of fatty alcohols.

12. A hair treatment composition according to any of the preceding claims, wherein the aqueous phase (I) and the oil phase (II) each have a viscosity of < 1000 mPas, preferably < 750 mPas, and most preferably < 500 mPas (measured at 20°C using a Brookfield DV-II viscometer, spindle 2, 20 rpm).

13. A method for conditioning hair, in which a hair treatment product according to any one of claims 1 through 12 is applied (preferably sprayed) to wet or dry hair after vigorous shaking and is left on the hair until the next hair wash.

14. Cosmetic use of a hair treatment product according to any one of claims 1 through 12 to improve
- wet and/or dry combability
- the feel of the hair all the way to the ends (in both wet and dry hair)
- the hair's texture
- the styleability / shaping of the hairstyle
- anti-frizz properties
- the smoothness of the hair
- the shine of the hair and/or
- static electricity in the hair.

## Revendications

1. Produit de soin capillaire sous la forme d'un système biphasique comportant deux phases continues distinctes, visuellement identifiables, séparées par une interface horizontale commune, contenant - par rapport au poids total du produit de soin capillaire - une phase aqueuse (I) dans une proportion d'au moins 85 % en poids et une phase huileuse (II) dans une proportion d'au plus 15 % en poids, dans lequel
- la phase huileuse (II) - par rapport à son poids total - est un mélange de
i) 20 à 40 % en poids d'au moins une isoparaffine comportant 9 à 17 atomes de carbone,
ii) 45 à 65 % en poids d'au moins deux mono- et/ou diesters différents d'acides carboxyliques linéaires ou ramifiés, saturés ou insaturés en C₄à C₁₂, avec des mono- ou dialcools linéaires ou ramifiés, saturés ou insaturés en C₄à C₂₄, et
iii) 5 à 20 % en poids d'au moins une huile végétale, et
- les produits de soin capillaire sont exempts de composés de silicone.

2. Produit de soin capillaire selon la revendication 1, contenant
a) la phase aqueuse (I) à raison d'au moins 88 % en poids par rapport au poids total du produit de soin capillaire et
b) la phase huileuse (II) à raison d'au plus 12 % en poids par rapport au poids total du produit de soin capillaire.

3. Produit de soin capillaire selon l'une des revendications précédentes, contenant comme isoparaffine i) de l'isodécane, de l'isoundécane, de l'isododécane, de l'isotridécane, de l'isotétradécane ainsi que des mélanges de ceux-ci.

4. Produit de soin capillaire selon la revendication 3, contenant de l'isoundécane, de l'isododécane et/ou de l'isotridécane.

5. Produit de soin capillaire selon l'une des revendications précédentes, contenant, en tant qu'au moins deux esters différents, ii) des esters ramifiés, la ramification pouvant se situer dans le radical alcool ou dans le radical acide.

6. Produit de traitement capillaire selon la revendication 5, contenant
- au moins un polyester de néopentyl-polyol, de préférence un diester de néopentylglycol, et
- au moins un ester d'un acide carboxylique en C₅à C₈réticulé et d'un monoalcool en C₁₂à C₂₄, de préférence des esters de l'acide 2-éthylhexylcarboxylique et de l'alcool myristylique, cétylique et/ou stéarylique.

7. Produit de soin capillaire selon l'une des revendications précédentes, contenant comme huile végétale iii) de l'huile de noix de macadamia.

8. Produit de soin capillaire selon l'une des revendications précédentes, contenant en outre dans la phase aqueuse (I), par rapport à son poids total, 0,01 à 2,5 % en poids d'au moins un polymère cationique.

9. Produit de soin capillaire selon l'une des revendications précédentes, contenant en outre dans la phase aqueuse (I), par rapport à son poids total, 0,01 à 1,0 % en poids d'au moins un tensioactif cationique.

10. Produit de soin capillaire selon l'une des revendications précédentes, contenant dans la phase aqueuse (I), par rapport à son poids total, > 85 % en poids, de préférence > 90 % en poids et en particulier > 95 % en poids d'eau.

11. Produit de soin capillaire selon l'une des revendications précédentes, exempt d'alcools gras.

12. Produit de soin capillaire selon l'une des revendications précédentes, dans lequel la phase aqueuse (I) et la phase huileuse (II) présentent chacune une viscosité < 1 000 mPas, de préférence < 750 mPas et de manière particulièrement préférée < 500 mPas (mesurée à 20 °C à l'aide d'un viscosimètre Brookfield DV-II, broche 2, 20 tr/min).

13. Procédé de soin capillaire, dans lequel un produit de soin capillaire selon l'une des revendications 1 à 12 est appliqué (de préférence vaporisé) sur les cheveux humides ou secs après avoir été vigoureusement agité, puis laissé sur les cheveux jusqu'au prochain lavage.

14. Utilisation cosmétique d'un produit de soin capillaire selon l'une des revendications 1 à 12 pour améliorer
- de la facilité de coiffage sur cheveux mouillés et/ou secs
- du toucher des cheveux jusqu'aux pointes (sur cheveux mouillés et secs)
- de la prise en main des cheveux
- de la facilité de coiffage / mise en forme de la coiffure
- des propriétés anti-frisottis
- la souplesse des cheveux
- la brillance des cheveux et/ou
- l'électricité statique des cheveux.
